# EUROPEAN PATENT SPECIFICATION

(11) **EP 0 994 185 B1**
(45) Date of publication and mention of the grant of the patent: **15.12.2004**
(21) Application number: 99308203.1
(22) Date of filing: 18.10.1999
(51) Int. Cl.: C12N 5/08, C12N 5/06

(54) **Method for culturing Langerhans islets cells**
Verfahren zur Kultivierung von Langerhansinsel-Zellen
Procédé de culture de cellules d'ilot de Langerhans

(30) Priority: 17.10.1998 KR 9843491
(43) Date of publication of application: 19.04.2000
(60) Divisional application: 03013538.8
(73) Proprietor: Yoon, Tai-Wook, Seodaemoon-Ku (KR)
(72) Inventor: Yoon, Tai-Wook, Seodaemoon-Ku (KR)
(74) Representative: Woods, Geoffrey Corlett

(56) References cited:
- EP-A- 0 363 125
- WO-A-82/00660
- WO-A-95/29989
- WO-A-97/16536
- HUOTARI MARI-ANNE ET AL: "Growth factor-mediated proliferation and differentiation of insulin-producing INS-1 and RINm5F cells: Identification of betacellulin as a novel beta-cell mitogen." ENDOCRINOLOGY, vol. 139, no. 4, April 1998 (1998-04), pages 1494-1499, XP000917303 ISSN: 0013-7227
- OBERG-WELSH CHARLOTTE ET AL: "Effects of vascular endothelial growth factor on pancreatic duct cell replication and the insulin production of fetal islet-like cell clusters in vitro" MOLECULAR AND CELLULAR ENDOCRINOLOGY,AMSTERDAM,NL, vol. 126, no. 2, 1997, pages 125-132, XP000864744 ISSN: 0303-7207

## Description

The present invention relates to a method for culturing the Langerhans islets suitable for transplantation. More particularly, the present invention relates to a culturing method by which the Langerhans islets can be proliferated in volume, and the fact that proliferated islet autotransplantation can stimulate islet regeneration via islet replication and neogenesis, leads to a perfect diabetes cure.

Diabetes mellitus (usually referred to simply as diabetes) is a complex disease characterized by a grossly abnormal pattern of carbohydrate metabolism resulting from impaired insulin secretion and/or effectiveness. The incidence of diabetes in industrialized countries is about 10%. Indeed, diabetes is the most common serious metabolic disease in the world; it affects hundreds of millions.

Diabetes may be classified as insulin-dependent diabetes or noninsulin-dependent diabetes. An absence of or insufficient intrinsic insulin is a characteristic of insulin-dependent diabetes. Some diabetics have a normal or even higher than normal level of insulin in their blood, but they are quite unresponsive to the hormone. This form of the disease, known as non-insulin-dependent diabetes, typically develops later in life than does the insulin-dependent form. However, the diabetes-causing mechanism with which these two types can be discriminated has yet to be revealed.

For treatment, insulin-dependent diabetics should continue to receive exogenous insulin because their capacity of producing insulin is greatly lowered. However, it is virtually impossible to continuously and properly provide insulin in response to patient's physiological demands. What is more difficult, the body has an insulin concentration gradient such that the insulin concentration is decreased in the order of the hepatic portal vein, the liver, the hepatic vein, the aorta and the muscle, but an injection of exogenous insulin does not result in such a concentration gradient, which then causes side effects.

The β- cells of the Langerhans islets secrete insulin and 11 other materials. Thus, an injection of only insulin can decrease the blood glucose level, but cannot prevent glucopenia and other complications. Since one of the objectives in the treatment of diabetes is to lower the blood glucose level, blood glucose lowering agents are often employed. These lowering agents, however, should not be prescribed for an extended period of time because they result in resistance. Moreover, blood glucose lowering agents were found to cause serious side effects.

Insulin, as mentioned above, is able to lower blood glucose level as well as gives much lower resistance than do blood glucose lowering agents. However, the necessary amount of insulin varies with a patient's conditions so that it is very difficult to timely administrate proper dosage of insulin. Upon improper administration of insulin, anti-insulin antibodies may be formed, making diabetes worse.

For curing diabetes, tissue transplantation has recently been of great interest. For example, the pancreas or Langerhans islets are transplanted into a patient who suffers from diabetes to provide a controlled amount of insulin which is necessary for the patient. EP-A-0 363 125 describes the culturing of human fetal pancreatic cells for implantation into diabetic patients.

In such cases, however, immune rejection is always problematic and must be considered. When the immune rejection occurs, immune suppressors are administered to the patients. In addition, the number of donors are not sufficient relative to the demand.

The treatment of diabetes by insulin administration was first conducted in 1921 by Banting and Beat, but they failed to cure the disease because of a diabetic complication. In 1966, Lillihei of Minnesota University first transplanted a portion of the pancreas into a diabetic patient. By 1977, 57 patients had been subjected to the transplantation. However, less than 10% of them survived for one year or more. Recent development of immune suppressors has increased the survival rate of pancreas or kidney transplant recipients up to 70%. For Langerhans islet transplantation, the survival rate amounts up to 90%.

The first thing into which account is taken is the histocompanibility between donor and recipient. If tissue transplantation is performed between two persons who have different histocompatibitity, an immune rejection occurs, leading to the destruction of the transplanted islets at the worst. Generally, 50 donors are needed to discover the necessary histocompatibility for one recipient. If fresh islets are transplanted, a large quantity of fibrous tissues grow out from freshly isolated Langerhans islets and divide and surround them if transplanted, so that the ability of the β-cells to secrete insulin in response to a stimulus declines greatly.

In approaching the present invention, the present inventors considered the following:

First, in order for cells or cell groups to proliferate in vitro, they musr contain stem cells or progenitor cells therein and be in undifferentiated states. Formunately, since many stem cells or progenitor cells exist in Langerhans islets, it is highly possible to proliferate undifferentiated Langerhans islets in vitro.

Next, MHC class II antigens, which cause immune rejection, must be absent in the proliferated Langerhans islets and thus, if the blood cells, rich in MHC class II antigens, are eliminated from Langerhans islets, the immune rejection can be greatly reduced in the islet allotransplantation. The longer the islets remain in the culture and proliferate, decreases the immune rejection response.

Finally, fibrous tissues are developed from the crude islets and must be able to be easily removed from the in vitro proliferated islet.

Taking advantage of the above three points, the present inventors tried to proliferate in vitro the Langerhans islets with the aim of preparing them so as to be easily and successfully transplanted in the host for the long term treatment of diabetes.

When the Langerhans islets were grown in a monolayer culture method, they proliferated at a rate of, at most, 80%. However, they poorly secreted insulin so that it was impossible to control the level of the blood glucose. The islets should proliferate at least 5-fold for successful transplantation.

Intensive and thorough research repeated by the present inventors resulted in the discovering that upon in vitro culture in media containing various biochemical materials, the Langerhans islets isolated from rats proliferate at high rates sufficient to be applied for transplantation, release the blood cells from themselves so as to greatly reduce the immune rejection, and function well enough so as to successfully continue to secrete insulin after transplantation according to the present invention.

Therefore, it is an object of the present invention, to provide a method for proliferating the Langerhans islets in a suitable state for transplantation, whereby a greatly enhanced treatment effect for diabetes can be brought about.

In accordance with the present invention, there is provided a method for proliferating the Langerhans islets, in which a culture medium is supplemented with radical scavengers, growth factors, a matrix material, nerve growth factor (NGF), cell migrating/scattering factors (such as hepatocyte growth factor (HGF)), antinecrosis factors or antiapoptosis factors insulin-like growth factor 1 (IGF 1), insulin-like growth factor 2 (IGF 2), vascular endothelial growth factor (VeGF) and a cytoskeleton activator (anti-integrin B1 antibody) at proper culture times and the proliferation is conduced for an extended period of time, so that the Langerhans islets are depleted of the blood cells and also proliferate sufficiently in order to be suitable for transplantation.

The present inventions are directed to a method for *in vitro* culturing and proliferating isolated Langerhans islets endocrine cells so as to be suitable for transplantation. The method comprises providing viable Langerhans islets endocrine cells including cells capable of differentiating into insulin producing cells and a first culturing medium comprising a basal medium supplemented with serum, at least one radical scavenger selected from the group consisting of nicotinamide, mannitol or superoxide dismutase; at least one growth factor selected from the group consisting of: insulin transferrin selenite-complex (ITS-complex), epidermal growth factor (EGF), platelet derived growth factor (PDGF), thrombin, Linoleic Acid-Bovine Serum Albumin (Linoleic Acid-BSA), hydrocortisone and progesterone; and at least one antinecrosis or antiapoptosis factor selected from the group consisting of: IGF 1, IGF2, VeGF and culturing the Langerhans islets endocrine cells including cells capable of differentiating into insulin producing cells for a period of about one day, for example 12 to 36 hours, in the first culturing medium to form a first culture growth. The first culture growth is collected and incubated at room temperature, in fresh Dulbeco modified Eagle medium (DMEM) or serum free basal medium with a cytoskeleton activator such as anti-integrin β1 antibody for about 45 to 120 minutes to form a second culture growth. The second culture growth is then suspended in a matrix material to provide a 3-dimensional culture growth environment and a second culturing medium comprising the supplemented basal medium and further including, at least, pituitary extract is added thereto and the culturing proceeds for about 1 or 2 days, for example 12 to 60 hours, to provide a third culture growth dispersed in the matrix material. A third culturing medium for culturing the third culture growth in the matrix material is provided and comprises the supplemented basal medium but without VeGF, and optionally adding to the third culturing medium NGF and HGF if the islets of the third culture growth appeared thick and the center of the islets appeared dark, or optionally adding to the third culturing medium NGF and anti-integrin β1 antibody if the islets appeared too spread out and then culturing for a period of about one or two days, for example 12 to 60 hours, to form a fourth culture growth. The islets are then collected from the matrix material placed in a suitable vessel and an enzyme, such as dispase, is added the collected islets and to loosen or enable removal of any adhering gel and then incubated, for example about 10 minutes, to provide an incubated product. The incubated product is then aspirated back and forth numerous times, for example at least 3 times such as 5 to 20 times, to cause the gel acted on by the enzyme to be removed from the islets thereby exposing the fibroblasts to the force created during the back and forth aspiration which appears to cause the fibroblasts to become separated from the surface of the islets to prepare fibroblast free islets.

The above process takes about 7 days and can be repeated as follows. A fourth culturing medium comprising the basal medium supplemented with, typically, serum, insulin-transferrin-sodium selenite (ITS), Linoleic Acid-BSA, thrombin, epidermal growth factor (EGF), nicotinamide, VeGF, IGF-1, IGF-2, superoxide dismutase and mannitol is provided and then the fibroblast free islets are cultured for about 8 to 12 hours to provide a fifth culture growth. The fifth culture growth is collected and cultured in a fifth culturing medium comprising DMEM an anti-integrin β1 antibody for 45 to 120 minutes at room temperature to form a sixth culture growth. The sixth culture growth is then suspended in a matrix material to provide a 3-dimensional culture growth environment and a sixth culturing medium comprising the supplemented basal medium and further including, at least, another growth factor is added thereto and then cultured for about 1 or 2 days, for example 12 to 60 hours, to provide a seventh culture growth dispersed in the matrix material. A seventh culturing medium which comprises the supplemented basal medium without VeGF, and optionally adding to the third culturing medium NGF and HGF if the islets of the third culture growth appeared thick and the center of the islets appeared dark, or optionally adding to the third culturing medium NGF and anti-integrin β1 antibody if the islets appeared too spread out is provided for culturing the seventh culture growth dispersed in the matrix material for a period of about one or two days, for example 12 to 60 hours, to form an eighth culture growth. The islets are then collected from the matrix material and an enzyme, such as diapase, is added to the collected islets and to any adhering gel and incubated, for example for about 10 min, to provide an incubated product. The incubated product is aspirated back and forth numerous times causing the gel acted on by the enzyme to be removed from the islets thereby exposing the fibroblasts, if any, to the force created during the back and forth aspiration causing the fibroblasts to become separated from the surface of the islets to prepare an increased number of fibroblast free islets.

In the present method it is preferred that the serum used in the medium is obtained from the same species as that of the langerhans islets to be proliferated. Thus, where the Langerhans islets are from a rat or human, the serum used is also rat, human serum, respectively, preferably the percent of serum in the medium is about 10%. The growth factor added to the second and sixth culturing medium is preferably pituitary extract.

The method of the present invention may be used to provide viable Langerhans islets endocrine cells including cells capable of differentiating into insulin producing cells for proliferation which are derived from a patient for proliferation which are then used for autotransplantation back into the same patient. This results in regeneration of the islets in the patient as described below.

The method of the invention may also include a method for removing fibroblasts growing from the surface of *in vitro* proliferated Langerhans islets by providing a plurality of proliferated islets having fibroblasts growing therewith in a gel matrix. The islets are collected from the gel matrix, along with the fibroblasts which are growing with the islets. An enzyme, such as dispase, is added to the collected islets and to any gel adhering to the surface of the collected islets and then this is incubated to provide an incubated product. The incubated product is then aspirated back and forth numerous times causing the gel acted on by the dispase to be removed from the islets thereby exposing the fibroblasts to the force being created during the back and forth aspiration which causes the fibroblasts to become separated from the surface of the islets to prepare fibroblast free islets.

The proliferared fibroblast free islets produced by the method according to the present invention may be used in treating diabetes mellitis by transplanting the proliferated Langerhans islets endocrine cells into a patient suffering from diabetes mellitis. In addition, the present invention further includes the use of the proliferated fibroblast free islets from a patient produced by the method according to the present invention to treat diabetes mellitis and to regenerate islets in the patent by autotransplanting the proliferated Langerhans islets endocrine cells into the patient suffering from diabetes mellitis.

The above and other objects and aspects of the invention will become apparent from the following description of embodiments with reference to the accompanying drawings in which:
Figure 1 is a photograph magnified 100 times (x100) showing blood cells released from islet after 24 hour incubation at 37°C;
Figure 2 is a photograph (x100) showing the Langerhans islets which are cultured in the absence of radical scavengers;
Figure 3 is a photograph (x100) showing the Langerhans islets which are cultured in the presence of a radical scavenger;
Figure 4 is a photograph (x200) showing islets which were incubated in a supplemented medium (VeGF, IGF-1, IGF-1);
Figure 5 is a photograph showing the islets on the second day of proliferation;
Figure 6 is a photograph (x100) showing the lateral growth of the Langerhans islets which are proliferated in the medium containing anti-integrin β1 antibody on the 3rd or 4th day;
Figure 7 is a photograph (x100) showing an islet cultured in the presence of migrating factors, for example HGF, showing horizontal growth or spreading of the islet, on the 3rd or 4th day;
Figures 8A and 8B are photographs (x320) showing the islet growing in all directions after proliferating for 10 days according to the method of the present invention;
Figure 9 is a photograph showing a human islet proliferated *in vitro* for a period of 8 days in the presence of human serum;
Figure 10 is a graph showing the change in the DNA amount of the Langerhans islets with culturing time in days;
Figures 11A, 11B and 11C show the process of fibroblast development and its depletion from the islets. Many fibroblasts grew out from islet during islet proliferation (Fig. 11A) even though the islets looked pure when they were collected. The fibroblasts were almost all removed (Fig. 11B) and completely removed (Fig. 11C) from the islets;
Figure 12 shows the *in vitro* functions (glucose-response) of fresh (square) and proliferated (circle) islets;
Figure 13 is a graph in which the levels of glucose in the blood of the diabetic rats which were transplanted with 1350 and 1650 islets just isolated, were measured and were plotted against time in days;
Figure 14 shows blood glucose level profile of Streptozotocin (STZ)-induced diabetic rat transplanted with 500 proliferated rat islets with days;
Figure 15 is a graph of blood glucose (mg/dl) levels against time in days comparing 500, 600, 800, 1000 and 1200 fresh rat islets transplanted into the spleen of STZ-induced diabetic mice, indicating that 800 islets are the minimum islet number required to recover and maintain normoglycaemia;
Figure 16 is a photograph (x200) of a fresh islet transplanted into the spleen of an SZT-induced diabetic nude mouse;
Figure 17 shows the blood glucose profile for 150 to 200 proliferated islets transplanted into the spleens of STZ-induced diabetic mice against time in days; and
Figure 18 is a photograph (x400) showing a proliferated islet transplanted into the spleen of STZ-induced diabetic nude mouse.

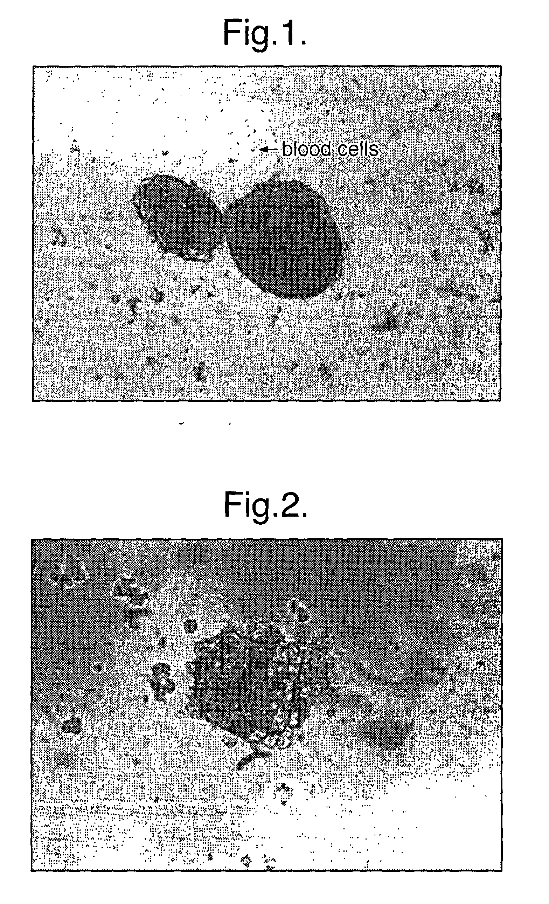

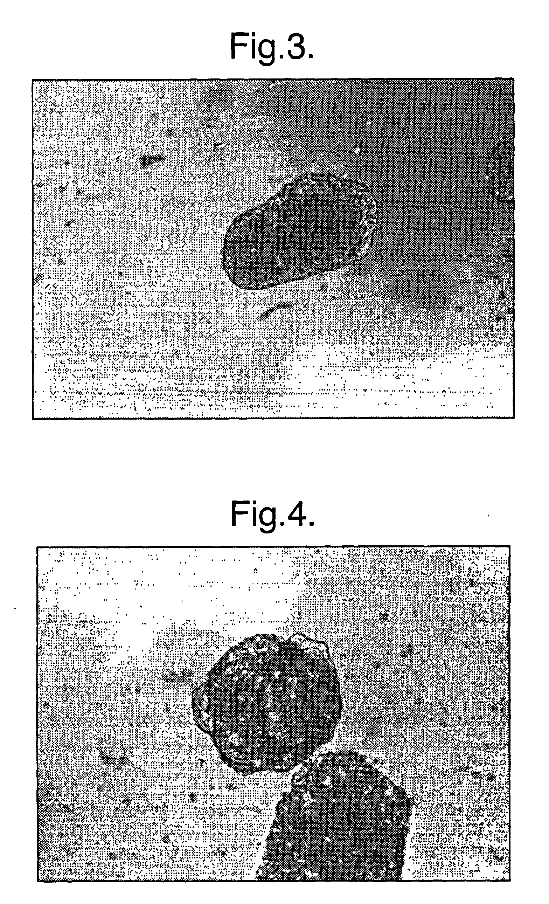

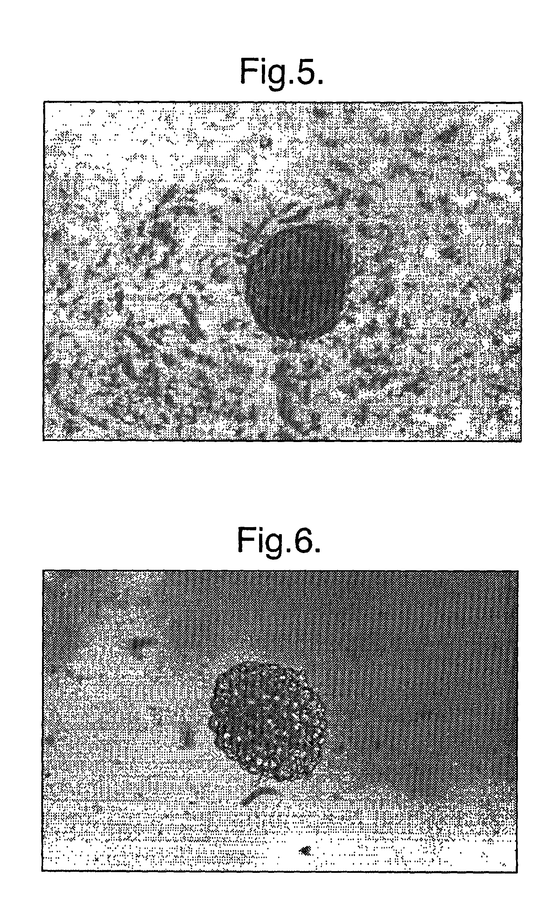

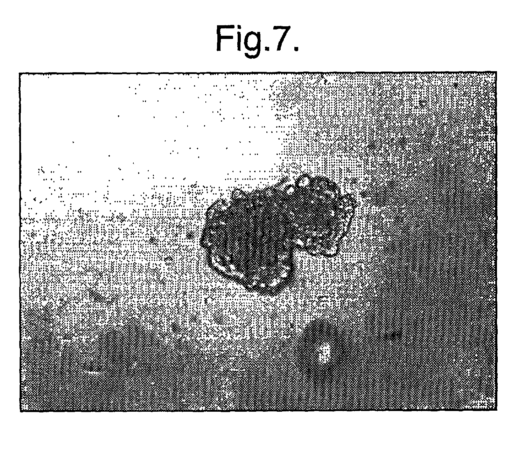

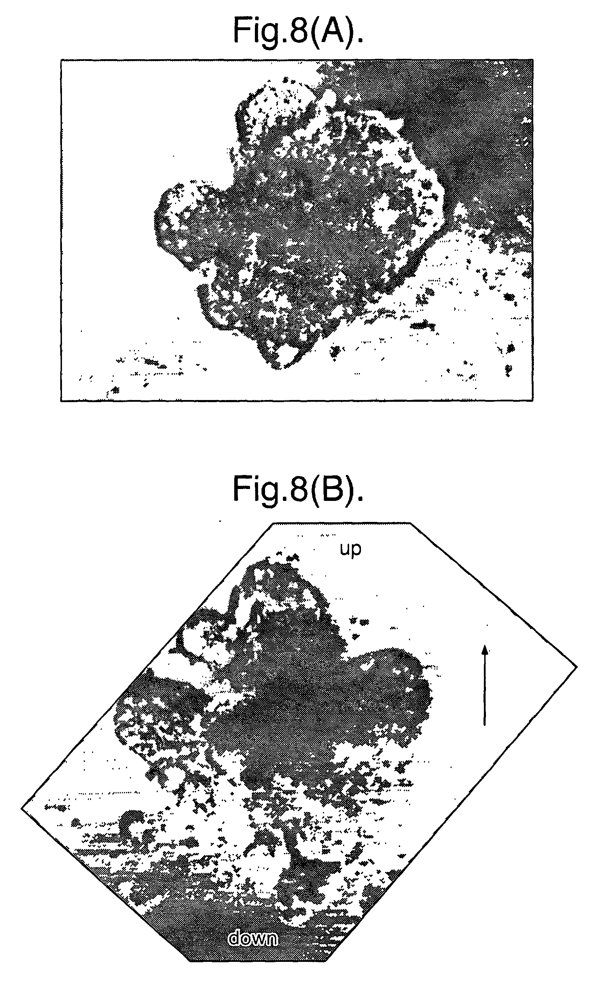

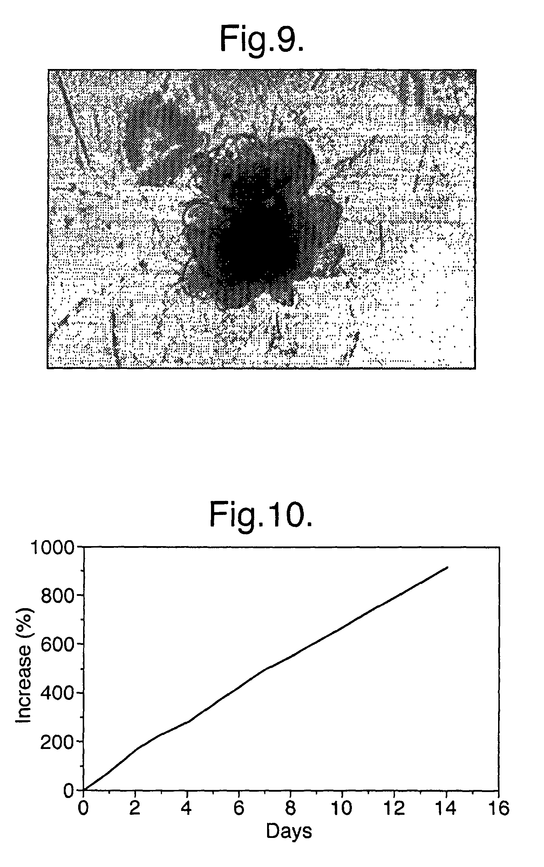

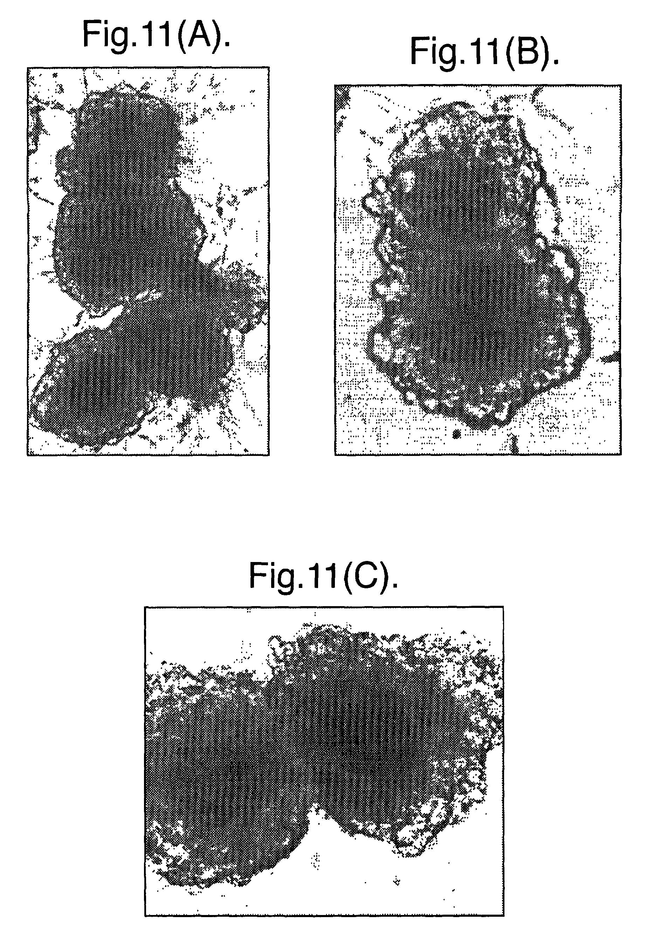

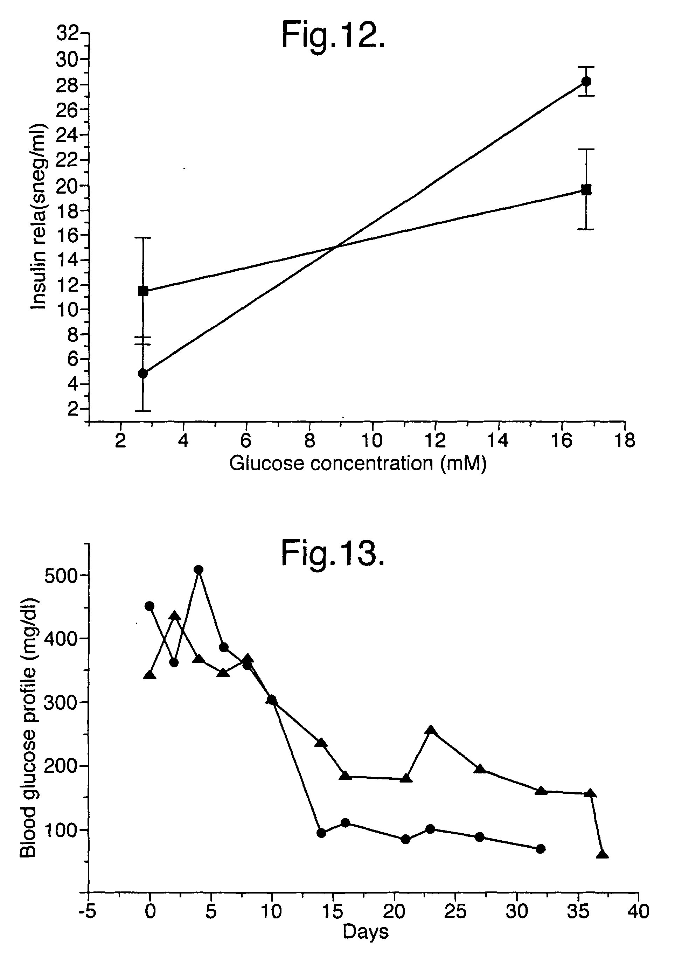

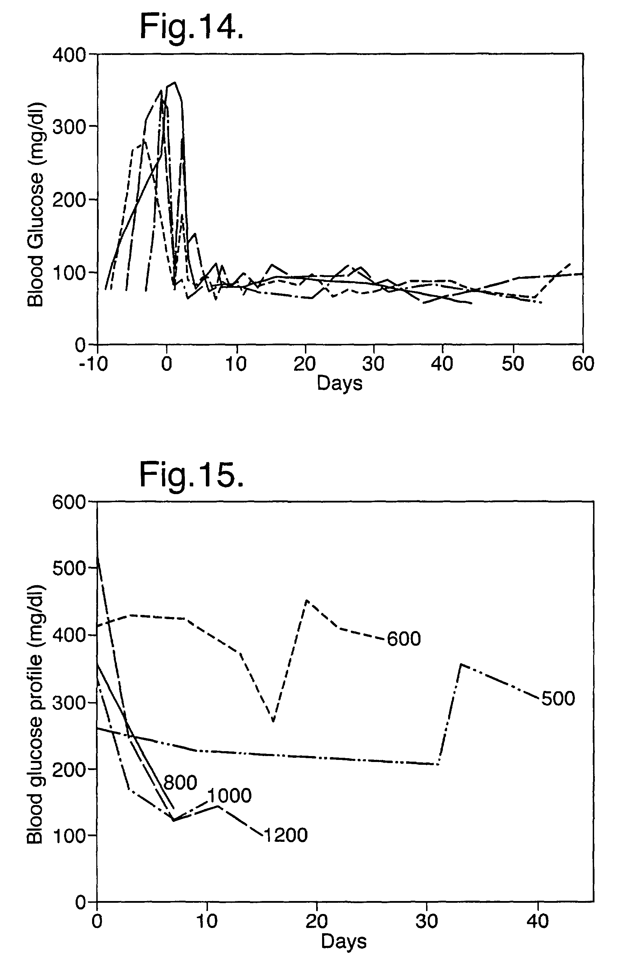

To better illustrate the present invention Figs. 1 through 18 are presented and described below. Fig. 1 shows the islet cells with released red blood cells after being incubated for 24 hours at 37°C in a medium of rat serum. Figs. 2 and 3 show the difference when culturing in the absence of and the presence of radical scavengers, respectively. Many islets cells were dead on the surface of the islet in the absence of a radical scavenger (Fig. 2). The islets look intact without dead cells on the surface in the presence of a radical scavenger (Fig. 3). Fig. 4 shows islets which were incubated in a medium supplemented with VeGF, IGF-1 AND IGF-2 to increase islet viability. The supplements function as anti-apoptotic and anti-necrotic factors. The islets are intact without dead cells on the surface. Fig. 5 is a photograph showing the islets on the second day of proliferation with the islets having a smooth surface and high viability. The medium used in Fig. 6 included anti-integrin β1 antibody and shows growing cells appearing on the surface of the islet, which then looks like a mulberry. Fig. 7 shows the effect of adding a migrating factor(s), for example hepatocyte growth factor (HGF), to the medium which appears to cause the islet to spread horizontally. Figs. 8A and 8B show that the medium used in the present invention enables the islets to grow in all directions. The front shape (Fig. 8A) and the lateral shape (Fig. 8B) show thick and long buds from the islet. Fig. 9 shows a human islet proliferated *in vitro* for 8 days using rat islet proliferation methods of the present invention. Fig. 10 shows the increase in DNA content *of in vitro* proliferating Sprague-Dawley (SD) rat islets against the number of days incubated. Figs. 11A shows fibroblasts growing out from pure islets during proliferation (x100 magnification), and Figs. 11B and 11C show the fibroblasts removed prior to transplantation (x200 magnification). Fig. 12 shows glucose-stimulated insulin secretion from freshly isolated (square) and proliferated (circle) islets. Fig. 13 is a graph of blood glucose (mg/dl) plotted against time in days for 1650 (circle) and 1350 (triangle) fresh rat islets transplanted into the liver of a STZ induced diabetic rat via the hepatic portal vein. Fig. 14 is a graph of blood glucose levels (mg/dl) plotted against time in days, 0 being the day of transplant of 500 proliferated rat islets into four (4) rats. Fig. 15 is a graph of blood glucose (mg/dl) levels plotted against time comparing 500, 600, 800, 1000 and 1200 fresh rat islets, respectively, transplanted into the spleen of STZ-induced diabetic mice. The numbers represent the number of islets transplanted islet. 800 islets are the minimum islet number required to recover and maintain normoglycaemia. The blood glucose levels of the diabetic rats which were transplanted with the Langerhans islets just isolated, were plotted against time in days.

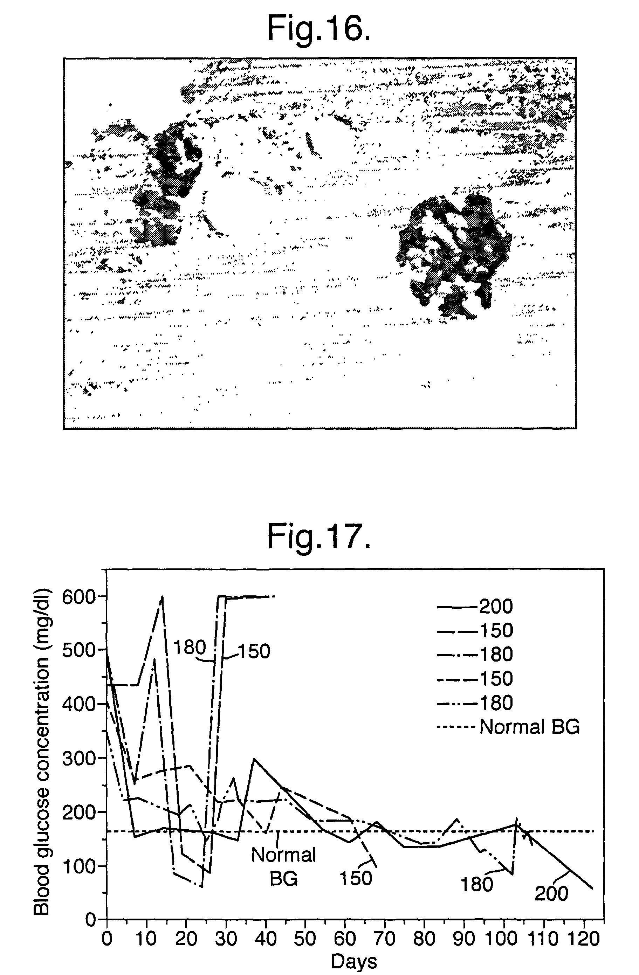

Fig. 16 is a photograph (x 200) of a fresh islet transplanted into the spleen of an SZT-induced diabetic nude mouse. The blood glucose concentration recovered and was maintained for 30 days. Then the mouse was sacrificed to collect transplanted islet. The islet was sectioned and stained in an insulin-specific staining method to identify the islet.

Fig. 17 is a graph of blood glucose levels (mg/dl) plotted against time in days comparing different numbers (200, 180, 150) of proliferated islets transplanted into spleens of STZ-induced diabetic mice. Blood glucose levels were measure every other day for several months. The mice used in this experiment had no working immune system, however, in two mice, one with an implanted islet number of 150 and 180, respectively, it is believed that the immune system functioned to the extent that it attacked the implanted islets resulting in the sudden increase in blood glucose level at around day 25.

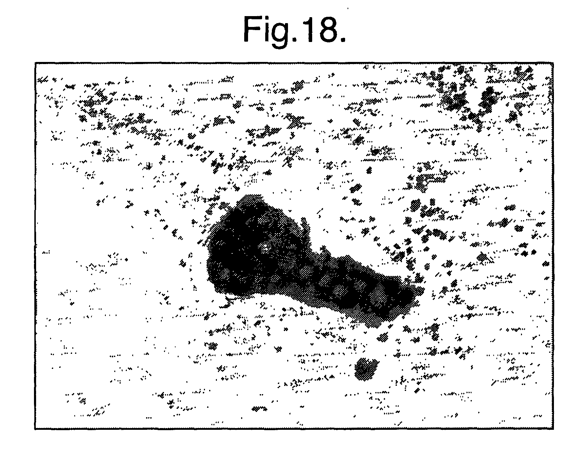

Fig. 18 is a photograph (x400) showing a proliferated islet transplanted into the spleen of STZ-induced diabetic nude mouse. The mouse recovered and remained normoglycaemia for 3 months. Then the mouse was sacrificed to remove the spleen from the mouse. The spleen was sectioned and stained in insulin-specific staining method to identify the transplanted islet.

Before culturing, the Langerhans islets isolated from the pancreas may be stored. Also, after culturing the proliferated Langerhans islets must be properly stored unless they all are used immediately. To this end, they are preferably frozen in liquid nitrogen.

Dimethyl sulfoxide (DMSO) is useful to protect the Langerhans islets upon freezing. At a convenient or proper time, the frozen stock of the Langerhans islets is thawed for culturing.

In accordance with the present invention, a culture system contains a matrix material in order to provide a three dimensional environment for Langerhans islets. Under this circumstance, the Langerhans islets show a high degree of proliferation. For the matrix material, collagen, complex collagen, tail complex collagen or other biogels (e.g. Matrigel®), or the like, may be used.

The culture medium of the present invention (rat serum for rat islets and human serum would be used for human islets) also contains a radical scavenger which plays the role of protecting the proliferated cells from radical damage. Nicotinamide, mannitol or a superoxide dismutase is added to the culture medium as a radical schvenger.

As mentioned above, the proliferation rate necessary for transplantation must amount to at least 500%. For this, the isolated Langerhans islets are cultured in the presence of growth factors, and cell migrating/scattering factors. Suitable growth factors are selected from the group consisting of insulin transferrin selenite (ITS), epidermal growth factor (EGF), platelet derived growth factor (PDGF), thrombin, progesterone, Linoleic Acid-BSA, pituitary extract and hydrocortisone. Examples of cell migrating/scattering factors include hepatic growth factor (HFG) and tumor promoting activator (TPA).

During culturing, the blood cells are go from the Langerhans islets into the medium. As the culturing goes on, the blood cells are subjected to necrosis. Since the blood cells have MHC class II, a major factor which causes the immune rejection upon tissue transplantation, the cultured Langerhans islets can be transplanted in a host with little immune rejection, if any. In the present invention, the cytoskeleton activator anti-integrin β1 antibody is added in the culture medium to enhance islet proliferation.

A better understanding of the present invention may be obtained in light of the following examples which are set forth to illustrate, but should not be construed to limit the present invention.

### EXAMPLE I : FREEZE STORAGE AND THAW OF LANGERHANS ISLETS

1 ml of 10% fetal calf (bovine) serum (FCS) medium which contained 2,000 (rat) Langerhans islets was added to 0.5 ml of 2M DMSO and allowed to stand at room temperature for 5 min. The procedure of adding DMSO and standing at room temperature was further repeated twice: for the first time, with 0.5 ml of 2M DMSO and a period of 25 min., and for the second time, with 0.5 ml of 3M DMSO and a period of 15 min. Thereafter, the medium was allowed to stand for 5 min on ice and then, for 15 min at -7.5 C. This medium was subjected to nucleation using previously chilled forceps and allowed to stand for 15 min at -7.5°C. The temperature was lowered down to -35 ~ - 40 ° C at a rate of 0.2 -0.3 per min. Once reaching the temperature, the vial containing the medium was stored in a liquid nitrogen tank.

For thawing, the vial stored in the liquid nitrogen tank was transferred to a cryovial in a water bath of 37°C until the ice crystals started to thaw. Then, the vial was placed on ice and allowed to stand in order that the Langerhans islets may settle on the bottom. The supernatant was drained out using a Pasteur pipette. The Langerhans islets were added with 10% FCS supplemented with 1 ml of 0.75 M sucrose and allowed to stand on ice for 30 min. Then, the Langerhans islets were added with 1 ml, 2 ml, 4 ml and 8 ml of 10% FCS, successively. After every addition, the Langerhans islets were allowed to stand for 5 min at room temperature. The resulting supernatant was removed and the remaining Langerhans islets were re-suspended in a culture medium and cultured at 37°C. This procedure may be used for rat, mouse, human, and the like, Langerhans islets.

### EXAMPLE II: PROLIFERATION OF THE LANGERHANS ISLETS

In this Example, the Langerhans islets which were frozen and thawed as in Example I were used. However, freshly isolated langerhans islets could also be used as is appreciated by one skilled in the art. During incubation 5% CO₂ is added to ambient air.

### Serum preparation

Rat serum was added to medium for proliferating the rat islets. 2-5 ml blood was obtained from a rat and transferred into sterile 15 ml Falcon tube. The blood was left for 2-4 hours at room temperature and then centrifuged at 3000g for 10 minutes. The supernatant was transferred to 1.5-2ml tubes, which were left overnight at 4°C and then centrifuged again in the same way. The supernatant was stored at -20°C until use. Human islets were proliferated in the medium containing 10% human serum instead of rat serum that was made in the same way.

The basal medium (50ml) is prepared by mixing together:

1.1 mg (100*µ*l) pyrubate, (Gibcobrl); 0.25*µ*g (100*µ*l) hydrocortisone (Sigma); 100 units/(100µl) (1ml) pencillin/streptomycin; 4.456mg (4µl) B-mercaptoethanol (Sigma); 14.6mg L-glutamate (Gibco); 238.3 (1ml) Hepes Buffer (Gibco); 100mg (11mM) glucose (Sigma) plus DMEM to make a volume of 50 ml.

### Experiment Example II-I: Culturing of Langerhans islets under basic conditions

First Day: 500 islets were cultured under basic conditions. The freshly isolated islets were incubated overnight at 37°C in 6 ml basal medium supplemented with 600 *µ*l rat serum (10%), 30*µ*g of insulin-transfenin-sodium selenite (ITS) (5*µ*g/ml, I-1884, Sigma USA), 6mg of Linoleic Acid-BSA (1mg/ml, L-8384, Sigma USA), 60ng (platelet-derived growth factor) PDGF (10ngm/ml P8147 Sigma USA), 600ng thrombin (100ngm/ml T-4393 Sigma USA), 60ngm (epidermal growth factor) EGF (10ngm/ml E-1264 Sigma USA), 187.2mg of 10⁻⁴ M superoxide dismutase (product no. S-9636, Sigma, USA), 109.32 *µ*g of 10⁻⁴ M mannitol (M-9546 Sigma USA), 61mg (3mM) nicotinamide (N-0636 Sigma USA), 12ng (vascular endothelial growth factor) VeGF (2ngm/ml V-7259 Sigma USA), 600ngm (insulin-like growth factor-I and -II) IGF-1 and IGF-2 (I-3769 and 1-213P, respectively, Sigma USA).

### Experiment Example II-II

### Second Day:

1. The 500 islets were incubated for 45 ~ 120 minutes at room temperature with 50ng (25ng/ml) of anti-itegrin B1 antibody (cat. No. I-41720 Transduction Labs. USA) in 2ml DMEM medium or serum free basal medium.
2. The islets were then suspended in 200µl of 80 to 100% Matrigel® (Collaborative Biomedical Product, USA) in order to provide a three-dimensional growth environment.
3. Added 2ml of the above supplemented basal medium and which was also supplemented with 100µg pituitary extract (50µg/ml P-1167 Sigma, USA) and cultured for 1-2 days at 37°C.

### Experiment Example II-III

### Third or Fourth Day:

The culture medium was replaced with fresh supplemented basal medium having the same composition as that of the first day except that VeGF is not added and, depending on the islet shape, other factors were added to the medium, as follows:
1. 20ng (nerve growth factor) NGF (10ng/ml N-6009 Sigma, USA), 50ng HGF (25ng/ml Collaborative Biomedical Product, USA Lot 902287), were added to 2ml of the supplemented basal medium if the islets became thick and dark in the center of the islet and then the islets were cultured in the medium for 1 or 2 days, or
2. 20ng NGF and 100ng anti-integrin β1 antibody (50ng/ml)) were added to 2 ml of the supplemented basal medium if the islets looked spread out and then cultured for 1 - 2 days.

Observation with a microscope at x200 magnification was shown in Figure 9.

After the Sixth or Seventh day, the islets culturing sequence was performed as follows:

### Seventh Day

The islets were collected from the 3-dimensional gel. The fibroblasts were removed using diapase by the process described below. The collected islets were cultured overnight in a floating culture, i.e. the islets were not fixed in a medium such as a gel medium. The culturing medium was 6 ml of the basal supplemented medium, as described above, supplemented with 600 µl of rat serum (10%), 30µg of insulin-transferrin-sodium selenite (ITS), 6mg of Linoleic Acid-BSA (1mg/ml), 600ng thrombin (100ngm/ml T-4393 Sigma USA), 60ng EGF, 101ng nicotinamide, 12ng VeGF, 600ng IGF-1, 600ng IGF-2, , 187.2mg of 10⁻⁴ M superoxide dismutase, (Sigma), 109.32 *µ*g of 10⁻⁴ M mannitol.

### Eight Day

1. The islets are incubated in fresh DMEM supplemented with 50ng of anti-itegrin B1 antibody for 45∼120 minutes at room temperature.
2. The islets were suspended in Matrigel® to grow in a 3-dimensional environment.
3. 2ml of the basal supplemented medium was added plus 100µg pituitary extract and were cultured for 1-2 days at 37°C.

### Ninth or Tenth Day

1. Basal supplemented medium added as first prepared, except that VeGF is not used.
2. Depending on the islet shape, the islets were cultured for 2 - 3 days in the above medium plus the added factors as described in the Third or Fourth Day culture medium.

### Fourteenth Day

1. The islets were collected from the gel by using dispase as described below and as done at around the Seventh day. This step is mainly to release the islets from the gel. However, if any fibroblasts were to remain, they also would be removed from the surface of the islet.
2. The islets were incubated at 37°C in a floating culture (islets not fixed as in a matrix) in a medium having the same constitutional amounts and components as in the 1^{st} day. These islets may be transplanted but are preferably subjected to removal as described below.

### Experiment Example III:

During culturing the amounts of DNA of the Langerhans islets were measured every other day and their relative amounts were plotted for culture times on the basis of the day on which the Langerhans islets were isolated. As seen in Fig. 10, the Langerhans islets proliferated up to about 1,000% on the 14th day after culturing.

### EXAMPLE IV : Test for in vivo function of the Langerhans Islets

### Experiment Example IV-1: Blood glucose level of diabetic rats transplanted with freshly isolated Langerhans islets.

For this, streptozotocin (STZ) was intra peritoneally injected to the rats at a dosage of 53-55 mg per kg of body weight and the level of glucose in the blood was checked everyday for two weeks. If the blood glucose level reached 250 ng/mg, the Sprague-Dawley (SD) rats were judged to be diabetic.

5.5 ml collagenase X1 (0.7mg/ml type X1 Sigma, USA) was introduced to the pancreases of healthy rats by injection via the common bile duct to distend the pancreases and to digest them for 17 min. at 37°C.

After the digested pancreas was washed three times with Dulbeco modified Eagle medium (DMEM), the Langerhans islets were isolated therefrom and collected in a discontinuous bovine serum albumin (BSA) concentration gradient method. The collected Langerhans islets were added with DMEM to a final volume of 100-150 *µ*l.

With the aid of 1 ml syringes, the resulting solution was injected into the hepatic portal vein of the diabetic rats anesthetized by intra peritoneal injection of Entobal (Hanlim Pharmacy Co. Ltd, Korea) at a dosage of 60 mg per kg body weight.

1650 (islet equivalents (IEQ) 2750) and 1350 (IEQ 2400) fresh Wistarkyoto (WK) rat islets were transplanted into the livers of 175g streptozotocin-induced diabetic rats via hepatic portal vein. 1650 fresh islets are enough mass to recover and maintain normoglycaemia but 1350 fresh islets are not enough mass to recover and maintain normolgycaemia, see Figure 13. Therefore, 1650 fresh islets are minimum required islet number.

### Experiment Example IV-II: Blood glucose level after transplantation of in vitro proliferated rat islets

The procedure of Experiment Example III-1 was repeated using the same Langerhans islets as those of Example II, which were proliferated in a medium supplemented with collagen, radical scavengers, cell migrating/scattering factors, growth factors and anti-integrin β1 antibody.

The blood glucose levels of the rat hosts transplanted with 500 proliferated rat islets were measured and plotted with times. The measurements of the blood glucose level are shown in Figure 14. As seen, 500 proliferated Langerhans islets completely functioned to recover and maintain normoglycaemia, so that enough insulin was secreted in proper response to the blood glucose levels of the hosts.

### Experiment Example IV-III:

Blood glucose concentration profile of STZ-induced diabetic mice transplanted with different number of fresh rat islets.

Different numbers of fresh rat islets were transplanted into spleen of STZ- induced diabetic nude mice to determine minimum number of islets required to recover and maintain normoglycaemia. The blood glucose levels of the most mice were measured and plotted wisth respect to time. The results are given in Figure 15. The figure shows the profile of blood glucose levels against time in days. At least 800 fresh islets are required to recover and maintain normoglycaemia.

The blood glucose level profile of STZ-induced diabetic mice of Figure 15 is based on STZ-induced diabetic mice transplanted with proliferated rat islets.

The Langerhans islets isolated from Sprague-Dawley (SD) rats were proliferated in the same medium as in Example II.

About 150 - 200 proliferated rat islets were transplanted into the spleen of STZ induce diabetic nude mice. The 150 - 200 proliferated rat islets are sufficient in number to recover and maintain normoglycaemia.

The islets were transplanted into the spleen of STZ-induced diabetic nude mice. The blood glucose levels of the host mice were measured and plotted against time in days. The results are given in Figure 17. As compared with Figure 15, in vitro proliferated (for 5-6 days) islets showed 3-5 fold higher in vivo function than fresh islets.

As described hereinbefore, the Langerhans islets, whether they are just isolated from pancreas or thawed from a frozen state, can be proliferated in volume upon culturing in a species similar serum (i.e. rat serum or human serum for rat or human Langerhans islets, respectively) medium further supplemented with radical scavenger, collagen, growth factors and cell migrating/scattering factors. Further, the Langerhans islets which are cultured for a long time in the supplemented medium, are depleted of blood cells, so that they can be transplanted and function well enough to recover and maintain normoglycaemia.

### Experiment Example V: In vitro function (glucose response) of proliferated islets.

Fresh and proliferated rat islets were incubated with 2.7 and 16.7 mM glucose for 1 hour and secreted insulin concentration were measured by using 125I-insulin KIT.

Proliferated islets respond nearly null to low glucose concentration and very strongly to high glucose concentration; whereas, fresh islets respond higher to low glucose, lower to high glucose concentration than proliferated islets (as see Fig 12). The results indicated that in vitro proliferated islets have more desirable functional properties than fresh islets, therefore proliferated islets can be used as autotransplantation material.

### Experiment Example VI: Fibroblast removal

Fresh islets are likely to be contaminated with fibroblasts even though they are collected in a pure state as can be seen by the fact that many fibroblasts grow out from the islets during the proliferation. The fibroblasts are removed completely prior to islet transplantation, as seen in Fig. 11C. Islets are dispersed in a gel (eg. Matrigel®) during proliferation. At about the seventh or fourteenth day, the islets are collected from the gel and 400 µl of dispase (Collaborative Biomedical Products, USA, Cat. 40235) is added and incubated for about 10 minutes at 37°C. Then the islets are aspirated back and forth several times causing the gel acted on by the diapase to be removed from the islets which exposes the fibroblasts to the force created during the back and forth aspiration causing the fibroblasts to become separated from the surface of the islets to prepare fibroblast free islets.

Islets proliferated up to 5- and 10-fold for the first and second week during *in vitro* culturing, respectively. These proliferated islets showed a more desirable *in vitro* glucose-response pattern than fresh islets. Their transplantation results showed that those islets have a 3~4-fold higher capability to recover and maintain normoglycaemia than fresh islets. These data suggest that *in vitro* proliferated islets are a better source than fresh islets for transplantation to treat diabetes. Consequently, the present invention can produce a large quantity of the Langerhans islets and, thus, transplantation using the proliferated islets according to the present invention is a promising therapeutic means for the treatment of diabetes.

### Experiment Example VII: Islet autotransplantation-stimulated islet regeneration.

Islet autotransplantation recovered and maintained normoglycaemia. In the diabetic rats, islet neogenesis did appear. Exogenous insulin injection also did not stimulate islet neogenesis. However, islet autotransplanation stimulates islet neogenesis.

The following table shows insulin content, number, size of islets collected from rats and mice which were either pancreatectomized (columns 2 and 3) or transplanted into diabetic mice/rats after transplantation (columns 4, 5 and 6). The islets used were either fresh (columns 4 and 5) or cultured/proliferated for 6 days (column 6) according to the process of the present invention. The transplantation (TX) was islet auto-transplantation. The results in columns 5 and 6 indicate that the transplanted islets, either fresh or proliferated according to the present invention result in islet regeneration in the pancreas.

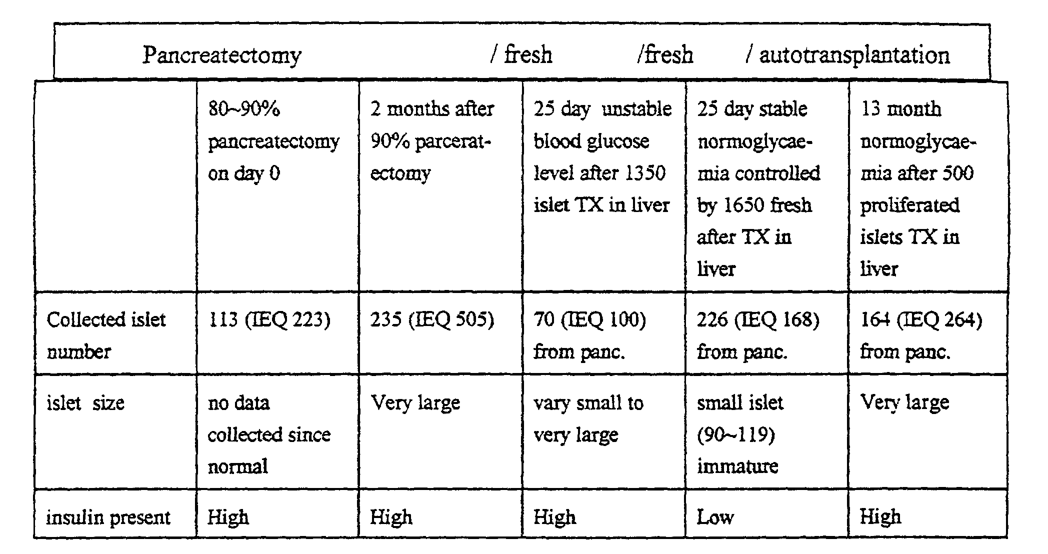

The following experimental results prove this (see Table, above)
1. 1350 fresh rat islets were transplanted into the liver of a STZ-induced diabetic rat via hepatic portal vein. Blood glucose concentration declined gradually to be normoglycaemia 35 days later. The values lingered in the 200's for 20 days and finally normoglycaemia for 1-2 days, as seen in Fig. 13. Islets were collected from the pancreas and stained deeply with dithizone.
2. 1650 fresh rat islets were transplanted syngeneically into the liver of a STZ-induced diabetic rat. The rat recovered normoglycaemia and maintained it for 25 days, as seen in Fig. 13. After sacrificing, 226 (IEQ 168) small islets were collected and were not stained with dithizone. The pancreas islets were immature and could be produced by neogenesis. In conclusion, on comparing both cases, islet autotransplantation-recovered normoglycaemia seems to provide a favorable environment for islet neogenesis and replication by secreting various natural substances.
3. The rats transplanted syngeneically with 500 proliferated islets maintained normoglycaemia for 13 months, as see Fig. 14. 164 large islets (IEQ 264) were collected and stained deeply with dithizone. The results indicates that neogenic (pancreas) islets grew up to large mature islets over a long period of time and islet autotransplantation enhances islet regeneration and stimulates islet maturation.
4. 90% pancreatectomy rat maintained normoglycaemia for 2 months. 235 large islets were collected from the remaining pancreas. The islets were stained deeply with dithizone. They seemed to originate from islet regeneration and grew for 2 months.
5. 90% pancreatectomy rat maintained normoglycaemia for 2 days. Islets were collected from the remaining pancreas.
6. In 90% pancreatectomy rat, 113 (IEQ 223) islets ere collected from the remaining head part of the pancreas on the day of the pancretectomy. On the basis of the pancreatectomy results, islet regeneration rate and size depends on the magnitude of islet deficiency.

Islet proliferation research is performed for the ultimate purpose of treating a diabetic patient by islet autotransplantation by providing a sufficient number of islets for transplant. The present invention provides the required number of islet since it enables successful islet proliferation *in vitro.* That is, one of the major reasons for the failure of transplantation is an insufficient number of islets are available for transplant into a diabetic patient. This problem is overcome by the present invention.

While the present invention was developed using rat and mice islets, the application of the present invention to proliferate human islets is within the scope of the teachings of the present invention, as can be appreciated by those skilled in the art.

## Claims

1. A method for *in vitro* culturing and proliferating isolated Langerhans islets endocrine cells so as to be suitable for transplantation, which method comprises:
(a) providing viable Langerhans islets endocrine cells including cells capable of differentiating into insulin producing cells;
(b) providing a first culturing medium comprising a basal medium supplemented with serum; at least one radical scavenger selected from nicotinamide, mannitol and superoxide dismutase; at least one growth factor selected from insulin transferrin selenite (ITS), epidermal growth factor (EGF), platelet derived growth factor (PDGF), thrombin, linoleic Acid-BSA, hydrocortisone and progesterone; and at least one antinecrosis or antiapoptosis factor selected from insulin-like growth factor-1 (IGF 1) and -II (IGF 2) and vascular endothelial growth factor (VeGF);
(c) culturing the said Langerhans islets endocrine cells for a period of about one day in the first culturing medium to form a first culture growth;
(d) collecting the first culture growth and incubating at about room temperature in fresh Dulbeco modified Eagle medium (DMEM) or serum free basal medium with anti-integrin β1 antibody for 45 to 120 minutes to form a second culture growth;
(e) suspending the second culture growth in a matrix material to provide a 3-dimensional culture growth environment, adding a second culturing medium comprising a said supplemented basal medium and further including, at least, pituitary extract, and culturing for 1 to 2 days to provide a third culture growth dispersed in the matrix material;
(f) providing a third culturing medium for culturing the third culture growth in the matrix material, the third culturing medium comprising a said supplemented basal medium without vascular endothelial growth factor (VeGF), and optionally adding to the third culturing medium nerve growth factor (NGF) and hepatocyte growth factor (HGF) if the islets of the third culture growth appeared thick and the center of the islets appeared dark or optionally adding to the third culturing medium (NGF) and anti-integrin β1 antibody if the islets appeared too spread out and culturing for a period of about one to two days to form a fourth culture growth;
(g) collecting the islets from the matrix material, adding an enzyme to the collected islets and to any adhering gel and incubating; and
(h) aspirating the incubated product to cause the gel acted on by the enzyme to be removed from the islets, thereby exposing the fibroblasts to the force created during the aspiration causing the fibroblasts to become separated from the surface of the islets to prepare fibroblasts-free islets.

2. A method according to claim 1 wherein the serum is obtained from the same species as that of the Langerhans islets.

3. A method according to claim 2 wherein the Langerhans islets are either from a rat and the serum used is about 10% rat serum or from a human and the serum used is about 10% human serum.

4. A method according to any one of the preceding claims wherein the said Langerhans islets endocrine cells in step (a) are derived from a patient for autotransplantation.

5. A method according to any one of the preceding claims further comprising:
(i) providing a fourth culturing medium comprising the basal medium supplemented with serum, insulin-transferrin-sodium selenite (ITS), Linoleic Acid-BSA, thrombin, EGF, nicotinamide, VeGF, IGF-1, IGF-2, superoxide dismutase and mannitol;
(j) culturing the fibroblast-free islets for about 8 to 12 hours to provide a fifth culture growth;
(k) providing a fifth culturing medium comprising DMEM with an integrin β1 antibody and culturing the fifth culture growth for 45 to 120 minutes at room temperature to form a sixth culture growth;
(l) suspending the sixth culture growth in a matrix material to provide a 3-dimensional culture growth environment, adding a sixth culturing medium comprising a said supplemented basal medium and further including, at least, pituitary extract, and culturing for 1 to 2 days to provide a seventh culture growth dispersed in the matrix material;
(m) providing a seventh culturing medium for culturing the seventh culture growth dispersed in the matrix material, the seventh culturing medium comprising the supplemented basal medium without vascular endothelial growth factor (VeGF), and optionally adding to the seventh culturing medium nerve growth factor (NGF) and hepatocyte growth factor (HGF) if the islets of the seventh culture growth appeared thick and the center of the islets appeared dark or optionally adding to the seventh culturing medium NGF and anti-integrin β1 antibody if the islets appeared too spread out and, culturing for a period of about one to two days to form an eighth culture growth; and
(n) collecting the islets from the matrix material, adding an enzyme to the collected islets and to any adhering gel and incubating;
(o) aspirating the incubated product to cause the gel acted on by the enzyme to be removed from the islets, thereby exposing the fibroblasts to the force created during the aspiration causing the fibroblasts to become separated from the surface of the islets to prepare an increased number of fibroblast-free islets.

## Patentansprüche

1. Verfahren zur in vitro-Züchtung und -Vermehrung von isolierten Langerhans-Inseln-Endokrinzellen, die für Transplantationszwecke geeignet sind, wobei das Verfahren folgendes umfasst:
(a) lebensfähige Langerhans-Inseln-Endokrinzellen unter Einschluss von Zellen, die zur Differenzierung zu Insulin erzeugenden Zellen befähigt sind, werden bereitgestellt;
(b) ein erstes Kulturmedium wird bereitgestellt, das folgendes umfasst: ein mit Serum ergänztes Grundmedium; mindestens einen Radikalfänger, der unter Nicotinamid, Mannit und Superoxid-dismutase ausgewählt ist; mindestens einen Wachstumsfaktor, der unter Insulin-Transferin-Selenit (ITS), epidermalem Wachstumsfaktor (EGF), von Blutplättchen abgeleiteter Wachstumsfaktor (PDGF), Thrombin, Linolsäure-BSA, Hydrocortison und Progesteron ausgewählt ist; und mindestens einen Antinekrose- oder Antiapoptosefaktor, der unter insulinartigem Wachstumsfaktor-1 (IGF 1) und -II (IGF 2) und vaskulärem endothelialem Wachstumsfaktor (VeGF) ausgewählt ist;
(c) die Langerhans-Inseln-Endokrinzellen werden für einen Zeitraum von etwa 1 Tag im ersten Kulturmedium gezüchtet, um ein erstes Kulturwachstum zu erzeugen;
(d) das erste Kulturwachstum wird gewonnen und etwa bei Raumtemperatur in frischem Dulbeco-modifiziertem Eagle-Medium (DMEM) oder serumfreiem Grundmedium mit anti-Integrin-β1-Antikörper 45 bis 120 Minuten gezüchtet, um ein zweites Kulturwachstum zu erzeugen;
(e) das zweite Kulturwachstum wird in einem Matrixmaterial suspendiert, um eine dreidimensionale Kulturwachstumsumgebung bereitzustellen, ein zweites Kulturmedium wird zugesetzt, das das ergänzte Grundmedium und ferner mindestens Hypophysenextrakt enthält, und eine 1- bis 2-tägige Züchtung wird durchgeführt, um ein drittes Kulturwachstum, das im Matrixmaterial dispergiert ist, zu erzeugen;
(f) ein drittes Kulturmedium zur Züchtung des dritten Kulturwachstums im Matrixmaterial wird bereitgestellt, wobei das dritte Kulturmedium das ergänzte Grundmedium ohne vaskulären endothelialen Wachstumsfaktor (VeGF) enthält, und gegebenenfalls wird das dritte Kulturmedium mit Nerven-Wachstumsfaktor (NGF) und Hepatozyten-Wachstumsfaktor (HGF) versetzt, wenn die Inseln des dritten Kulturwachstums ein dickes Erscheinungsbild zeigen und das Zentrum der Inseln ein dunkles Erscheinungsbild zeigt, oder das dritte Kulturmedium wird mit (NGF) und anti-Integrin-β1-Antikörper versetzt, wenn die Inseln ein zu stark verteiltes Erscheinungsbild zeigen, und die Züchtung wird für eine Zeitspanne von etwa 1 bis 2 Tagen durchgeführt, um ein viertes Kulturwachstum zu erzeugen;
(g) die Inseln aus dem Matrixmaterial werden gewonnen, ein Enzym wird zu den gewonnenen Inseln und zu etwaigem haftendem Gel zugegeben und eine Inkubation wird durchgeführt; und
(h) das inkubierte Produkt wird abgesaugt, um eine Entfernung des Gels, auf das das Enzym eingewirkt hat, von den Inseln zu bewirken, wodurch die Fibroblasten der während des Absaugvorgangs erzeugten Kraft ausgesetzt werden, was bewirkt, dass die Fibroblasten von der Oberfläche der Inseln unter Bildung von fibroblastenfreien Inseln getrennt werden.

2. Verfahren nach Anspruch 1, wobei das Serum von der gleichen Spezies wie die Langerhans-Inseln erhalten wird.

3. Verfahren nach Anspruch 2, wobei die Langerhans-Inseln von einer Ratte stammen und es sich beim verwendeten Serum um etwa 10 % Rattenserum handelt, oder von einem Menschen stammen und es sich beim verwendeten Serum um etwa 10 % Humanserum handelt.

4. Verfahren nach einem der vorstehenden Ansprüche, wobei die Langerhans-Inseln-Endokrinzellen in Stufe (a) von einem Patienten für eine Autotransplantation stammen.

5. Verfahren nach einem der vorstehenden Ansprüche, ferner umfassend:
(i) ein viertes Kulturmedium wird bereitgestellt, das das Grundmedium umfasst, das mit Serum, Insulin-Transferin-Natriumselenit (ITS), Linolsäure-BSA, Thrombin, EGF, Nicotinamid, VeGF, IGF-1, IGF-2, Superoxid-dismutase und Mannit ergänzt ist;
(j) die fibroblastenfreien Inseln werden etwa 8 bis 12 Stunden gezüchtet, um ein fünftes Kulturwachstum zu erzeugen;
(k) ein fünftes Kulturmedium wird bereitgestellt, das DMEM mit einem Integrin-β1-Antikörper umfasst, und das fünfte Kulturwachstum wird 45 bis 120 Minuten bei Raumtemperatur gezüchtet, um ein sechstes Kulturwachstum zu erzeugen;
(l) das sechste Kulturwachstum wird in einem Matrixmaterial suspendiert, um eine dreidimensionale Kulturwachstumsumgebung bereitzustellen, ein sechstes Kulturmedium wird zugesetzt, das das ergänzte Grundmedium und ferner mindestens Hypophysenextrakt enthält, und eine 1- bis 2-tägige Züchtung wird durchgeführt, um ein siebtes Kulturwachstum, das im Matrixmaterial dispergiert ist, zu erzeugen;
(m) ein siebtes Kulturmedium zur Züchtung des siebten Kulturwachstums, das im Matrixmaterial dispergiert ist, wird bereitgestellt, wobei das siebte Kulturmedium das ergänzte Grundmedium ohne vaskulären endothelialen Wachstumsfaktor (VeGF) umfasst, und gegebenenfalls wird das siebte Kulturmedium mit Nervenwachstumsfaktor (NGF) und Hepatozytenwachstumsfaktor (HGF) versetzt, wenn die Inseln des siebten Kulturmediums ein dickes Erscheinungsbild zeigen und das Zentrum der Inseln ein dunkles Erscheinungsbild zeigt, oder gegebenenfalls wird das siebte Kulturmedium mit NGF und anti-Integrin-β1-Antikörper versetzt, wenn die Inseln ein zu stark verteiltes Erscheinungsbild zeigen, und die Züchtung wird für eine Zeitspanne von 1 bis 2 Tagen durchgeführt, um ein achtes Kulturwachstum zu erzeugen; und
(n) die Inseln aus dem Matrixmaterial werden gewonnen, ein Enzym wird zu den gewonnenen Inseln und zu etwaigem haftenden Gel zugegeben und eine Inkubation wird durchgeführt;
(o) das inkubierte Produkt wird abgesaugt, um eine Entfernung des Gels, auf das das Enzym eingewirkt hat, von den Inseln zu bewirken, wodurch die Fibroblasten der während des Absaugevorgangs erzeugten Kraft ausgesetzt werden, was bewirkt, dass die Fibroblasten von der Oberfläche der Inseln unter Bildung einer erhöhten Anzahl von fibroblastenfreien Inseln getrennt werden.

## Revendications

1. Procédé pour cultiver *in vitro* et faire proliférer des cellules endocrines isolées d'îlots de Langerhans, de façon à ce qu'elles soient appropriées à la transplantation, ce procédé comprenant :
(a) le fait de fournir des cellules endocrines viables d'îlots de Langerhans incluant des cellules capables de se différencier en cellules produisant de l'insuline ;
(b) le fait de fournir un premier milieu de culture comprenant un milieu de base supplémenté avec du sérum ; au moins un piégeur de radicaux choisis parmi la nicotinamide, le mannitol et la superoxyde dismutase ; au moins un facteur de croissance choisi parmi l'insuline-transferrine-sélénite (ITS), le facteur de croissance épidermique (EGF), le facteur de croissance dérivé des plaquettes (PDGF), la thrombine, l'acide linoléique-BSA, l'hydrocortisone et la progestérone ; et au moins un facteur antinécrotique ou antiapoptotique choisi parmi le facteur de croissance analogue à l'insuline 1 (IGF-1) et II (IGF-2) et le facteur de croissance endothélial vasculaire (VeGF) ;
(c) le fait de cultiver lesdites cellules endocrines d'îlots de Langerhans pendant une période d'environ un jour dans le premier milieu de culture pour former une première croissance de culture ;
(d) le fait de collecter la première croissance de culture et de l'incuber à environ température ambiante dans du milieu d'Eagle modifié par Dulbeco (DMEM) frais ou du milieu de base sans sérum avec un anticorps anti-intégrine ß1 pendant 45 à 120 minutes pour former une seconde croissance de culture ;
(e) le fait de mettre en suspension la seconde croissance de culture dans un matériau matrice pour obtenir un environnement de croissance de culture en trois dimensions, d'ajouter un deuxième milieu de culture comprenant un dit milieu de base supplémenté et comprenant, en outre, au moins un extrait pituitaire, et de cultiver pendant 1 ou 2 jours pour obtenir une troisième croissance de culture dispersée dans le matériau matrice ;
(f) le fait de fournir un troisième milieu de culture pour cultiver la troisième croissance de culture dans le matériau matrice, le troisième milieu de culture comprenant un dit milieu de base supplémenté sans facteur de croissance endothélial vasculaire (VeGF) et d'ajouter éventuellement au troisième milieu de culture du facteur de croissance du tissu nerveux (NGF) et du facteur de croissance des hépatocytes (HGF) si les îlots de la troisième croissance de culture semblent épais et le centre des îlots apparaît sombre, ou d'ajouter éventuellement au troisième milieu de croissance du NGF et de l'anticorps anti-intégrine ß1 si les îlots semblent trop éparpillés et de les cultiver pendant une période d'environ un à deux jours pour former une quatrième croissance de culture ;
(g) le fait de collecter les îlots à partir du matériau matrice, d'ajouter une enzyme aux îlots collectés et à tout gel adhérant et de les incuber ; et
(h) le fait d'aspirer le produit incubé pour provoquer l'élimination du gel sur lequel a agi l'enzyme des îlots, et d'exposer ainsi les fibroblastes à la force créée pendant l'aspiration en provoquant la séparation des fibroblastes de la surface de îlots pour préparer des îlots sans fibroblastes.

2. Procédé selon la revendication 1, dans lequel le sérum est obtenu à partir de la même espèce que celle des îlots de Langerhans.

3. Procédé selon la revendication 2, dans lequel les îlots de Langerhans proviennent d'un rat et le sérum utilisé est du sérum de rat à environ 10 %, ou proviennent d'un humain et le sérum utilisé est du sérum humain à environ 10 %.

4. Procédé selon l'une quelconque des revendications précédentes, dans lequel lesdites cellules endocrines d'îlots de Langerhans dans l'étape (a) sont dérivées d'un patient pour une autotransplantation.

5. Procédé selon l'une quelconque des revendications précédentes, comprenant en outre :
(i) le fait de fournir un quatrième milieu de culture comprenant le milieu de base supplémenté avec du sérum, de l'insuline-transferrine-sélénite de sodium (ITS), de l'acide linoléique-BSA, de la thrombine, de l'EGF, de la nicotinamide, du VeGF, de l'IGF-1, de l'IGF-2, de la superoxyde dismutase et du mannitol ;
(j) le fait de cultiver les îlots sans fibrobastes pendant environ 8 à 12 heures pour obtenir une cinquième croissance de culture ;
(k) le fait de fournir un cinquième milieu de culture comprenant du DMEM avec un anticorps intégrine ß1 et de cultiver la cinquième croissance de culture pendant 45 à 120 minutes à température ambiante pour former une sixième croissance de culture ;
(l) le fait de mettre en suspension la sixième croissance de culture dans un matériau matrice pour obtenir un environnement de croissance de culture en trois dimensions, d'ajouter un sixième milieu de culture comprenant un dit milieu de base supplémenté et comprenant, en outre, au moins un extrait pituitaire, et de cultiver pendant 1 ou 2 jours pour obtenir une septième croissance en culture dispersée dans le matériau matrice;
(m) le fait de fournir un septième milieu de culture pour cultiver la septième croissance de culture dispersée dans le matériau matrice, le septième milieu de culture comprenant le milieu de base supplémenté sans facteur de croissance endothélial vasculaire (VeGF), et d'ajouter éventuellement au septième milieu de croissance du facteur de croissance du tissu nerveux (NGF) et du facteur de croissance des hépatocytes (HGF) si les îlots de la septième croissance de culture semblent épais et que le centre des îlots apparaît sombre, ou d'ajouter éventuellement au septième milieu de croissance du NGF et de l'anticorps anti-intégrine ß1 si les îlots semblent trop éparpillés et de les cultiver pendant une période d'environ un à deux jours pour former une huitième croissance de culture ; et
(n) le fait de collecter les îlots à partir du matériau matrice, d'ajouter une enzyme aux îlots collectés et à tout gel adhérant et de les incuber ;
(o) le fait d'aspirer le produit incubé pour provoquer l'élimination du gel sur lequel a agi l'enzyme, des îlots, et d'exposer ainsi les fibroblastes à la force créée pendant l'aspiration en provoquant la séparation des fibroblastes de la surface des îlots pour préparer un nombre accru d'îlots sans fibroblastes.
